# EUROPEAN PATENT SPECIFICATION

(11) **EP 4 477 246 B1**
(45) Date of publication and mention of the grant of the patent: **14.10.2026**
(21) Application number: 22925820.7
(22) Date of filing: 08.02.2022
(51) Int. Cl.: A61M 25/10, A61M 25/00

(54) **BALLOON CATHETER AND METHOD OF MANUFACTURING BALLOON CATHETER**
BALLONKATHETER UND VERFAHREN ZUR HERSTELLUNG EINES BALLONKATHETERS
CATHÉTER À BALLONNET ET PROCÉDÉ DE FABRICATION DE CATHÉTER À BALLONNET

(43) Date of publication of application: 18.12.2024
(73) Proprietor: ASAHI INTECC CO., LTD., Seto-shi, Aichi 489-0071 (JP)
(72) Inventor: KATOU Taishi, Seto-shi, Aichi 489-0071 (JP)
(74) Representative: Winter, Brandl - Partnerschaft mbB
(86) International application number: PCT/JP2022/004905
(87) International publication number: WO 2023/152798

(56) References cited:
- EP-A1- 1 051 990
- EP-A1- 3 733 236
- EP-B1- 2 398 541
- WO-A1-2019/131158
- CN-B- 105 536 121
- JP-B2- 4 854 458
- US-A1- 2010 318 113
- US-A1- 2012 150 107
- US-A1- 2012 150 107

## Description

### TECHNICAL FIELD

The technique disclosed herein relates to a balloon catheter, and a method of manufacturing the balloon catheter.

### BACKGROUND ART

Balloon catheters are elongated medical appliances that are inserted into a body lumen such as a blood vessel to widen and expand a constricted part in the body lumen, or to restrict stream of a fluid (e.g. blood flow) by obstructing the body lumen.

A balloon catheter includes an inner layer tube, an outer layer that covers an outer peripheral surface of the inner layer tube, and a balloon that covers an outer peripheral surface of the outer layer (e.g. refer to WO 2020/075278 A1, EP 3 733 236 A1, US 2012/150107 A). An inner cavity of the inner layer tube is in communication with an inner space of the balloon to serve as an expansion medium lumen. When the balloon is expanded, an expansion medium (contrast medium, physiological saline, etc.) is fed toward the inner space of the balloon through the inner cavity of the inner layer tube as the expansion medium lumen. When the balloon is contracted, the expansion medium is discharged from the inner space of the balloon through the inner cavity of the inner layer tube as the expansion medium lumen.

### SUMMARY OF INVENTION

### TECHNICAL PROBLEM

In balloon catheters, a negative pressure is generated in an expansion medium lumen when the expansion medium is discharged through the expansion medium lumen to contract the balloon. In the conventional balloon catheter configurations, the inner layer tube may be detached from the outer layer and crushed by the negative pressure generated in the expansion medium lumen, and the conventional balloon configurations have room for improvement in terms of smoothening discharge of the expansion medium through the expansion medium lumen.

In this specification, a technique capable of solving the above-described problems is disclosed. The problem is solved by the subject-matters of the independent claims. Further aspects are disclosed in the dependent claims.

### SOLUTION TO PROBLEM

The technique disclosed in this specification can be achieved e.g. as the following aspects.

(1) The balloon catheter disclosed in this specification includes a first inner layer tube, a second inner layer tube, an outer layer, a balloon, and an adhesive layer. The outer layer covers the outer peripheral surface of the first inner layer tube and an outer peripheral surface of the second inner layer tube. The balloon covers the outer peripheral surface of the outer layer. The inner space of the balloon and the inner cavity of the first inner layer tube are in communication with each other. The adhesive layer is placed between the first inner layer tube and the outer layer.

In this balloon catheter, the inner cavity of the inner layer tube is in communication with the inner space of the balloon to serve as an expansion medium lumen. The inner layer tube is bonded to the outer layer via the adhesive layer. Thus, if a negative pressure is generated in the expansion medium lumen due to discharge of the expansion medium through the expansion medium lumen, the inner layer tube can be prevented from being detached from the outer layer and crushed. Thus, this balloon catheter makes it possible to suppress decrease in a cross-sectional area of the expansion medium lumen due to the detachment and crushing of the inner layer tube and to achieve smooth discharge of the expansion medium through the expansion medium lumen.

(2) The balloon catheter is configured such that the adhesive layer has a hardness lower than any of hardness of at least a part of the outer layer and hardness of at least a part of the first inner layer tube. This configuration can improve the bonding between the inner layer tube and the outer layer via the adhesive layer. Thus, according to this configuration, detachment of the inner layer tube from the outer layer can be effectively suppressed, and the expansion medium can be more smoothly discharged through the expansion medium lumen.

(3) The balloon catheter may be configured such that the inner layer tube has a hardness higher than that of the outer layer. This configuration can effectively suppress crushing of the inner layer tube if a negative pressure is generated in the expansion medium lumen. Thus, according to this configuration, crushing of the inner layer tube can be effectively suppressed, and the expansion medium can be more smoothly discharged through the expansion medium lumen.

(4) The balloon catheter may be configured such that the outer layer includes a first outer layer and a second outer layer placed on a proximal end side relative to the first outer layer, and the first outer layer has a hardness lower than that of the second outer layer. According to this configuration, a distal end portion of the balloon catheter can be made relatively softer for improving its followability for a body lumen, while a proximal end portion of the balloon catheter can be made relatively harder for improving its pushability.

(5) The balloon catheter may be configured such that the adhesive layer has a hardness lower than hardnesses of all of the first outer layer, the second outer layer, and the inner layer tube. According to this configuration, the adhesive layer can improve the bonding of the inner layer tube to both the first and second outer layers. Thus, according to this configuration, detachment of the inner layer tube from the outer layer including the first and second outer layers can be effectively suppressed, and the expansion medium can be more smoothly discharged through the expansion medium lumen.

(6) The balloon catheter may be configured such that the inner layer tube has a hardness higher than hardnesses of both the first outer layer and the second outer layer. This configuration makes it possible to effectively suppress crushing of the inner layer tube if a negative pressure is generated in the expansion medium lumen. Thus, according to this configuration, the crushing of the inner layer tube can be more effectively suppressed, and the expansion medium can be more smoothly discharged through the expansion medium lumen.

(7) In a method of manufacturing a balloon catheter disclosed in this specification, the balloon catheter includes an inner layer tube, a second inner layer tube, an outer layer covering an outer peripheral surface of the first inner layer tube and an outer peripheral surface of the second inner layer tube, and a balloon covering an outer peripheral surface of the outer layer and having an inner space in communication with an inner cavity of the first inner layer tube. The method includes: preparing the first inner layer tube and disposing a material for an adhesive layer on an outer peripheral surface of the inner layer tube; fabricating a precursor by covering the first inner layer tube with a material for the outer layer; and forming the outer layer by heating the precursor to melt the material for the outer layer and furthermore forming the adhesive layer between the inner layer tube and the outer layer by melting the material for the adhesive layer. The adhesive layer has a hardness lower than any of hardness of at least a part of the outer layer and hardness of at least a part of the first inner layer tube. The method of manufacturing the balloon catheter can facilitate manufacture of the balloon catheter with the inner layer tube bonded to the outer layer via the adhesive layer.

The technique disclosed in this specification can be achieved in various aspects e.g. in aspects of a balloon catheter and a manufacture method thereof.

### BRIEF DESCRIPTION OF DRAWINGS

FIG. 1 is an explanatory view illustrating a side configuration of a balloon catheter 100 according to the first embodiment.
FIG. 2 is an explanatory view illustrating a transverse sectional configuration of the balloon catheter 100 according to the first embodiment.
FIG. 3 is an explanatory view illustrating a longitudinal sectional configuration of the balloon catheter 100 according to the first embodiment.
FIG. 4 is an explanatory diagram presenting a hardness of each member constituting the balloon catheter 100 according to the first embodiment.
FIG. 5 is an explanatory view illustrating an example of a method of manufacturing the balloon catheter 100 according to the first embodiment.
FIG. 6 is an explanatory view illustrating an example of a method of manufacturing the balloon catheter 100 according to the first embodiment.
FIG. 7 is an explanatory view illustrating an example of a method of manufacturing the balloon catheter 100 according to the first embodiment.
FIG. 8 is an explanatory view illustrating a side configuration of a balloon catheter 100a according to the second embodiment.
FIG. 9 is an explanatory diagram presenting a hardness of each member constituting the balloon catheter 100a according to the second embodiment.
FIG. 10 is an explanatory view illustrating a longitudinal sectional configuration of the balloon catheter 100 according to a modification.

### EMBODIMENTS OF THE INVENTION

### A. First embodiment:

### A-1. Configuration of Balloon Catheter 100:

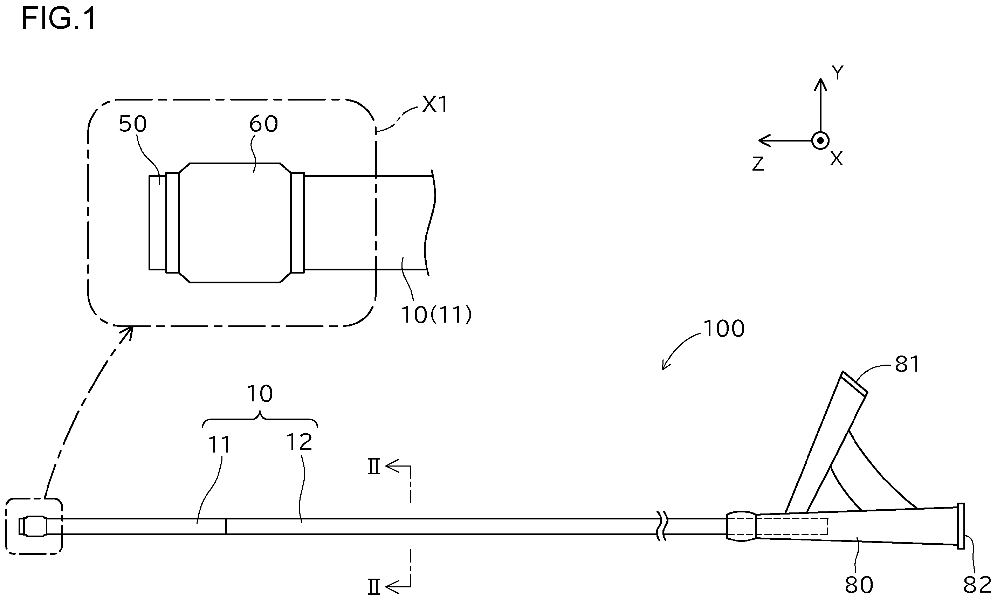

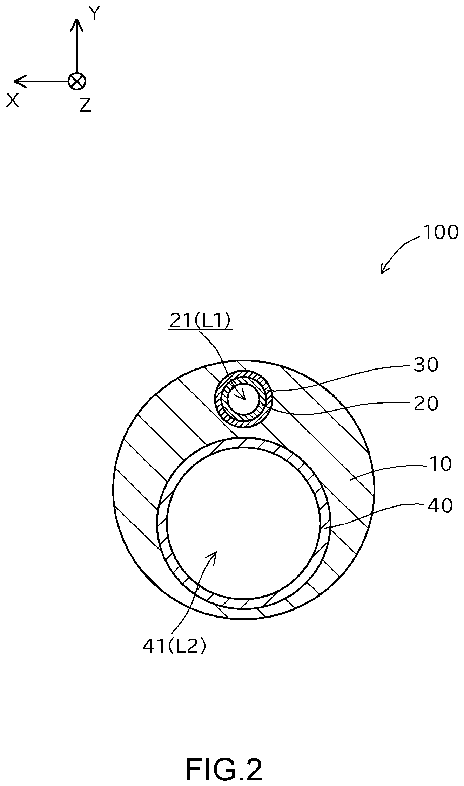

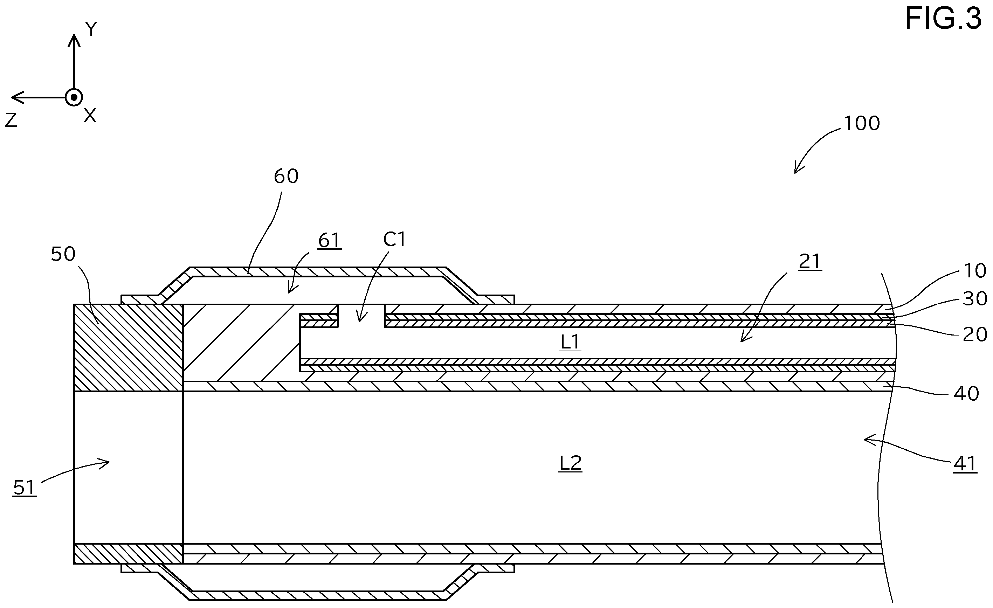

FIG. 1 to FIG. 3 are explanatory views illustrating a configuration of a balloon catheter 100 according to the first embodiment. FIG. 1 illustrates a side configuration of the balloon catheter 100, FIG. 2 illustrates a transverse sectional configuration of the balloon catheter 100, taken along II-II in FIG. 1, and FIG. 3 illustrates a longitudinal sectional configuration of a distal end portion (X1 portion in FIG. 1) of the balloon catheter 100. In some figures, illustration of a part of the balloon catheter 100 is omitted. In each figure, the positive side in Z-axis direction is defined as the distal end side (farther side) to be inserted into a body, and the negative side in Z-axis direction is defined as the proximal end side (nearer side) to be manipulated by a professional such as a surgeon. Hereinafter, in the balloon catheter 100 and its components, the end on the distal end side is referred to as a "distal end", the distal end together with its vicinity is referred to as a "distal end portion", the end on the proximal end side is referred to as a "proximal end", and the proximal end together with its vicinity is referred to as a "proximal end portion". In FIG. 1, the balloon catheter 100 is, as a whole, in a straight line shape parallel to Z-axis direction, but is flexible enough to be curved.

The balloon catheter 100 is an elongated medical appliance that is inserted into a body lumen such as a blood vessel to widen and expand a constricted part in the body lumen, or to restrict stream of a fluid (e.g. blood flow) by obstructing the body lumen. The total length of the balloon catheter 100 is e.g. about 1500 mm to 2000 mm.

The balloon catheter 100 according to the first embodiment is a balloon guiding catheter having a device lumen L2 described later for guiding a device. For example, in a procedure in which a thrombus in a cerebral blood vessel is removed using a thrombus removing catheter delivered through the device lumen L2, the balloon catheter 100 is used to stop the blood flow so that pieces of the stripped thrombus do not flow through the blood vessel.

The balloon catheter 100 includes an outer layer 10, a first inner layer tube 20, an adhesive layer 30, a second inner layer tube 40, a distal tip 50, a balloon 60, and a Y connector 80.

The first inner layer tube 20 is a cylindrical (e.g. hollow cylindrical) member. The first inner layer tube 20 has an outer diameter of e.g. about 0.08 mm to 0.8 mm, and a wall thickness of e.g. about 10 µm to 80 µm. An inner cavity 21 of the first inner layer tube 20 serves as an expansion medium lumen L1 through which an expansion medium (contrast medium, physiological saline, etc.) for expanding the balloon 60 flows. The first inner layer tube 20 is an example of the inner layer tube in claims.

The first inner layer tube 20 is made of a flexible material. Examples of the material for the first inner layer tube 20 include resins, more specifically polyamide, polyamide elastomer, polyester, polyurethane, polyurethane elastomer, and polyetheretherketone (PEEK).

The transverse sectional shape of the first inner layer tube 20 (an example of the inner layer tube in claims) can be set as appropriate. Examples of the transverse sectional shape of the first inner layer tube 20 include a circler shape, an oval shape, and a polygonal shape. Examples of the polygonal shape include a square shape and a rectangular shape. When the transverse sectional shape of the first inner layer tube 20 is e.g. oval or rectangular, the size along the radial direction of the oval or rectangular first inner layer tube 20 can be smaller than the size along a direction perpendicular to the radial direction. Such a size setting makes it possible to decrease the size along the radial direction of the first inner layer tube 20 when the area of the expansion medium lumen L1 is the same as of the first inner layer tube 20 in the transverse section of the first inner layer tube 20 (without decreasing the flow rate of the expansion medium), and therefore the balloon catheter 100 can be decreased in diameter.

The second inner layer tube 40 is a cylindrical (e.g. hollow cylindrical) member. The second inner layer tube 40 has an inner diameter of e.g. about 1.5 mm to 3.0 mm, and a wall thickness of e.g. about 5 µm to 30 µm. An inner cavity 41 of the second inner layer tube 40 serves as the device lumen L2 through which a guide wire or various devices (e.g. suction catheter or thrombus removing catheter) are inserted.

The second inner layer tube 40 is made of a flexible material. Examples of the material for the second inner layer tube 40 include resins, more specifically PTFE (polytetrafluoroethylene), PVDF (polyvinylidene fluoride), PFA (perfluoroalkoxy alkane), FEP (perfuluoroethylene propane), ETFE (ethylene/tetrafluoroethylene), polyethylene, and polypropylene. For example, a reinforcing body formed by knitting a metal wire into a mesh shape may be arranged so as to cover the outer peripheral surface of the second inner layer tube 40.

The outer layer 10 is a cylindrical member that covers the outer peripheral surface of the first inner layer tube 20 and the outer peripheral surface of the second inner layer tube 40. In this specification, the phrase "the A member covers the outer peripheral surface of the B member" means that the A member is placed outside at least a part of the outer peripheral surface of the B member, and does not exclude an aspect in which another member is interposed between the A member and the outer peripheral surface of the B member. In the first embodiment, the outer layer 10 covers almost the entire outer peripheral surface of the first inner layer tube 20 over almost the entire length of the first inner layer tube 20, and also covers almost the entire outer peripheral surface of the second inner layer tube 40 over almost the entire length of the second inner layer tube 40. The outer layer 10 has an outer diameter of e.g. about 2.0 mm to 3.5 mm.

The outer layer 10 is made of a flexible material. Examples of the material for the outer layer 10 include resins, more specifically polyamide, polyamide elastomer, polyester, polyurethane, and polyurethane elastomer.

In the first embodiment, the outer layer 10 includes a first outer layer 11, and a second outer layer 12 placed on the proximal end side relative to the first outer layer 11. The second outer layer 12 is connected to the proximal end portion of the first outer layer 11 so as to be substantially coaxial with the first outer layer 11. The first outer layer 11 has a hardness lower than that of the second outer layer 12.

The adhesive layer 30 is placed between the first inner layer tube 20 and the outer layer 10 to bond them. That means, the first inner layer tube 20 is bonded to the outer layer 10 via the adhesive layer 30. Examples of the material for the adhesive layer 30 include resins, more specifically urethane resins. The second inner layer tube 40 is directly joined to the outer layer 10 e.g. by fusion bonding.

The distal tip 50 is a cylindrical (e.g. hollow cylindrical) member. The distal tip 50 is placed on the distal end side of the outer layer 10 and joined to the distal end of the outer layer 10 e.g. by fusion bonding. The outer diameter of the distal tip 50 is substantially equal to that of the outer layer 10. An inner cavity 51 of the distal tip 50 is in communication with the inner cavity 41 of the second inner layer tube 40 and serves as the device lumen L2 together with the inner cavity 41 of the second inner layer tube 40. Examples of the material for the distal tip 50 include resins, more specifically urethane resins.

The balloon 60 is a hollow member having an inner space 61 and is expandable and contractable in association with feeding and discharge of the expansion medium. The balloon 60 covers the outer peripheral surface of the distal end portion of the outer layer 10 and the outer peripheral surface of the distal tip 50. The distal end portion of the balloon 60 is joined to the outer peripheral surface of the distal tip 50 e.g. by fusion bonding, and the proximal end portion of the balloon 60 is joined to the outer peripheral surface of the distal end portion of the outer layer 10 e.g. by fusion bonding.

Examples of the material for the balloon 60 include resins and rubbers, more specifically polyolefins such as polyethylene, polypropylene, polybutene, ethylenepropylene copolymer, ethylene-vinyl acetate copolymer, ionomer, and a mixture of two or more of them; thermoplastic resins such as soft polyvinyl chloride resin, polyamide, polyamide elastomer, polyester, polyester elastomer, polyurethane, and fluororesin; silicone rubbers; and latex rubbers.

As illustrated in FIG. 3, an inner space 61 of the balloon 60 and the inner cavity 21 (expansion medium lumen L 1) of the first inner layer tube 20 are in communication with each other. That means, the balloon catheter 100 has a communication channel (communication hole) C1 that extends radially from the inner peripheral surface on the distal end portion of the first inner layer tube 20 to the outer peripheral surface of the outer layer 10, and the inner space 61 of the balloon 60 and the inner cavity 21 of the first inner layer tube 20 are in communication with each other through the communication channel C1.

The Y connector 80 constitutes the proximal end portion of the balloon catheter 100. The Y connector 80 has an expansion medium port 81 in communication with the expansion medium lumen L1, and a device port 82 in communication with the device lumen L2. When expanding the balloon 60, the expansion medium is fed to the inner space 61 of the balloon 60 from the expansion medium port 81 through the expansion medium lumen L1 and the communication channel C1. When contracting the balloon 60, the expansion medium in the inner space 61 of the balloon 60 is discharged from the expansion medium port 81 through the communication channel C1 and the expansion medium lumen L1. Through the device port 82, the device is inserted into the device lumen L2 and drawn out from the device lumen L2. The balloon catheter 100 according to the first embodiment is a so-called over-the-wire (OTW) type balloon catheter.

### A-2. Hardness Relationship between Each Member:

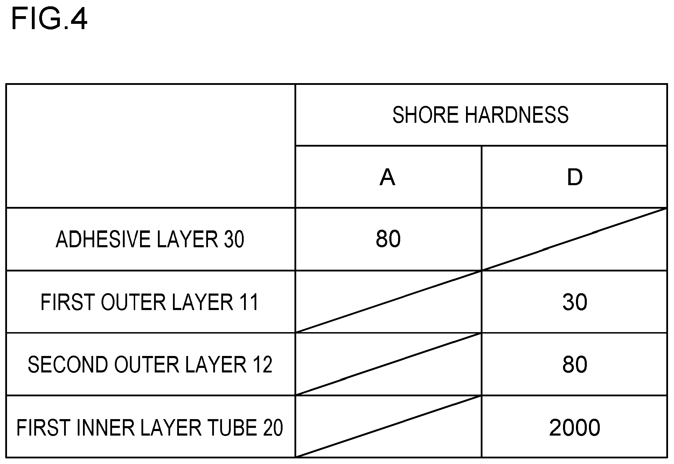

FIG. 4 is an explanatory diagram presenting a hardness of each member constituting the balloon catheter 100 according to the first embodiment. As illustrated in FIG. 4, in the balloon catheter 100 according to the first embodiment, the adhesive layer 30 has a shore hardness (A) of 80, the first outer layer 11 has a shore hardness (D) of 30, the second outer layer 12 has a shore hardness (D) of 80, and the first inner layer tube 20 has a shore hardness (D) of 2000. That means, in the balloon catheter 100 according to the first embodiment, the hardnesses of the four members are set in an ascending order of the adhesive layer 30, the first outer layer 11, the second outer layer 12, and the first inner layer tube 20. Such a hardness relationship can be achieved e.g. by forming the adhesive layer 30 from a urethane resin, the first outer layer 11 from a mixed material of a urethane resin and a nylon resin, the second outer layer 12 from a nylon resin, and the first inner layer tube 20 from PEEK.

In the balloon catheter 100 according to the first embodiment, since the hardness relationship between each member is as described above, the following relationships (1) to (5) are established.
(1) The adhesive layer 30 has a hardness lower than hardnesses of both at least a part of the outer layer 10 and at least a part of the first inner layer tube 20.
(2) The first inner layer tube 20 has a hardness higher than that of the outer layer 10.
(3) The first outer layer 11 has a hardness lower than that of the second outer layer 12.
(4) The adhesive layer 30 has a hardness lower than hardnesses of all of the first outer layer 11, the second outer layer 12, and the first inner layer tube 20.
(5) The first inner layer tube 20 has a hardness higher than hardnesses of both the first outer layer 11 and the second outer layer 12.

The above-described hardness level relationship between each member (adhesive layer 30, first outer layer 11, second outer layer 12, and first inner layer tube 20) can also be expressed as a relationship of melting point levels between materials for each member. That means, in the balloon catheter 100 according to the first embodiment, the melting points of the materials for the four members are set in an ascending order of the adhesive layer 30, the first outer layer 11, the second outer layer 12, and the first inner layer tube 20. For example, the adhesive layer 30 is made of a material with a melting point of 81°C, the first outer layer 11 is made of a material with a melting point of 170°C, the second outer layer 12 is made of a material with a melting point of 178°C, and the first inner layer tube 20 is made of a material with a melting point of 334°C.

In the first embodiment, since the melting points are significantly different between the first inner layer tube 20 and the outer layer 10 (the first outer layer 11 and the second outer layer 12), the first inner layer tube 20 and the outer layer 10 cannot be fusion-bonded to each other. For this reason, the first inner layer tube 20 and the outer layer 10 are bonded to each other via the adhesive layer 30. The second inner layer tube 40 is made of a material capable of fusion-bonding to the outer layer 10 and is fusion-bonded with the outer layer 10.

### A-3. Method of Manufacturing Balloon Catheter 100:

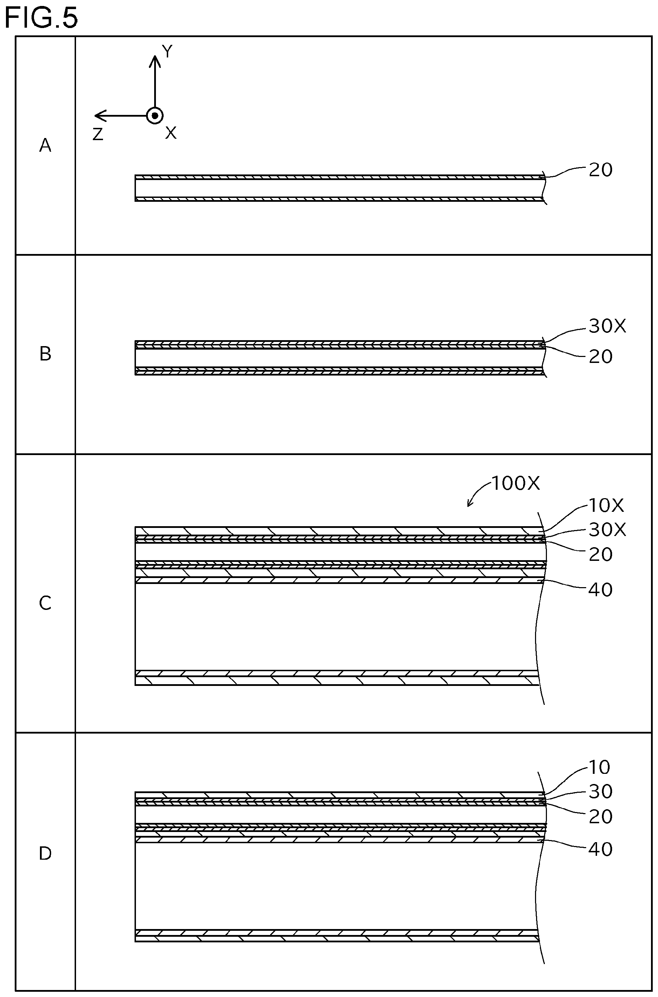

FIG. 5 to FIG. 7 are explanatory views illustrating an example of a method of manufacturing the balloon catheter 100 according to the first embodiment. In manufacturing the balloon catheter 100, the first inner layer tube 20 is first prepared as illustrated in column A of FIG. 5, and a material 30X for the adhesive layer 30 is placed on the outer peripheral surface of the first inner layer tube 20 as illustrated in column B of FIG. 5. The material 30X for the adhesive layer 30 can be placed on the outer peripheral surface of the first inner layer tube 20 e.g. by extrusion coating. The placement of the material 30X for the adhesive layer 30 may be performed concurrently with fabrication of the first inner layer tube 20 by coextrusion.

Next, as illustrated in column C of FIG. 5, a precursor 100X is fabricated by covering the first inner layer tube 20 and the separately prepared second inner layer tube 40 with a material 10X for the outer layer 10. In the first embodiment, the outer layer 10 includes the first outer layer 11 and the second outer layer 12, and therefore the material 10X for the outer layer 10 includes the material for the first outer layer 11 and the material for the second outer layer 12.

Subsequently, the precursor 100X is heated. This heating is performed at a temperature at which the material 30X for the adhesive layer 30 and the material 10X of the outer layer 10 are molten. By this heating, the material 10X for the outer layer 10 is molten to form the outer layer 10, and the material 30X for the adhesive layer 30 is molten to form the adhesive layer 30 that is placed between the first inner layer tube 20 and the outer layer 10 to bond them, as illustrated in column D of FIG. 5. At this time, the second inner layer tube 40 is fusion-bonded to the outer layer 10. This heating may be performed on the entire precursor 100X at once, or the precursor 100X may be divided into a portion made of the material for the first outer layer 11 and a portion made of the material for the second outer layer 12 so that these parts are sequentially subjected to the heating. For example, the portion made of the material for the first outer layer 11 in the precursor 100X may be first heated, followed by heating of the portion made of the material for the second outer layer 12.

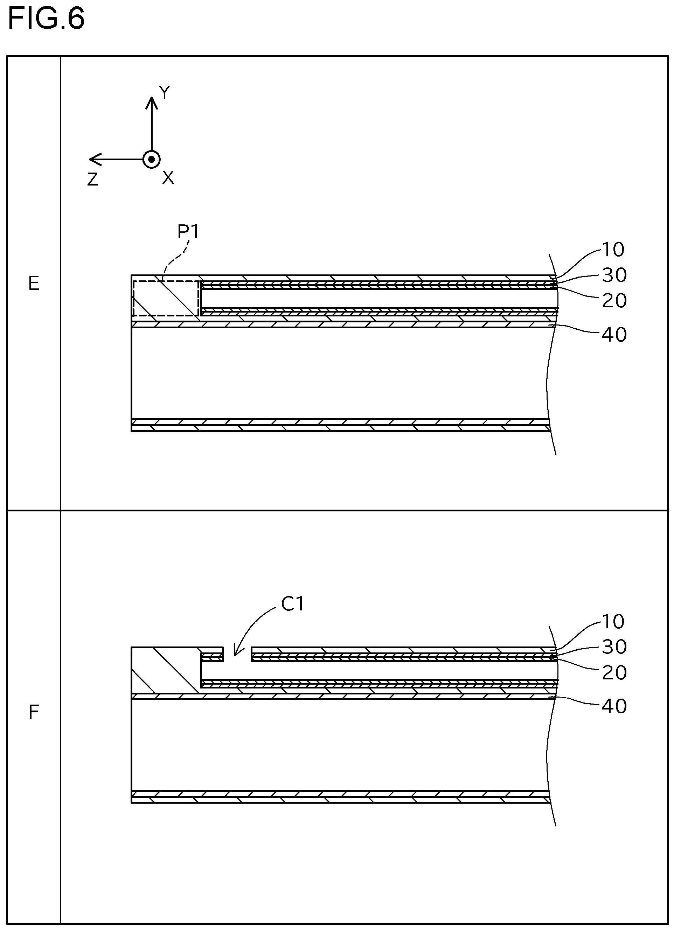

Subsequently, the distal end portion of the first inner layer tube 20 (portion P1 in the figure) is removed as illustrated in column E of FIG. 6, and the communication channel C1 that radially extends from the inner peripheral surface on the distal end portion of the first inner layer tube 20 to the outer peripheral surface of the outer layer 10 is formed e.g. by laser processing as illustrated in column F of FIG. 6.

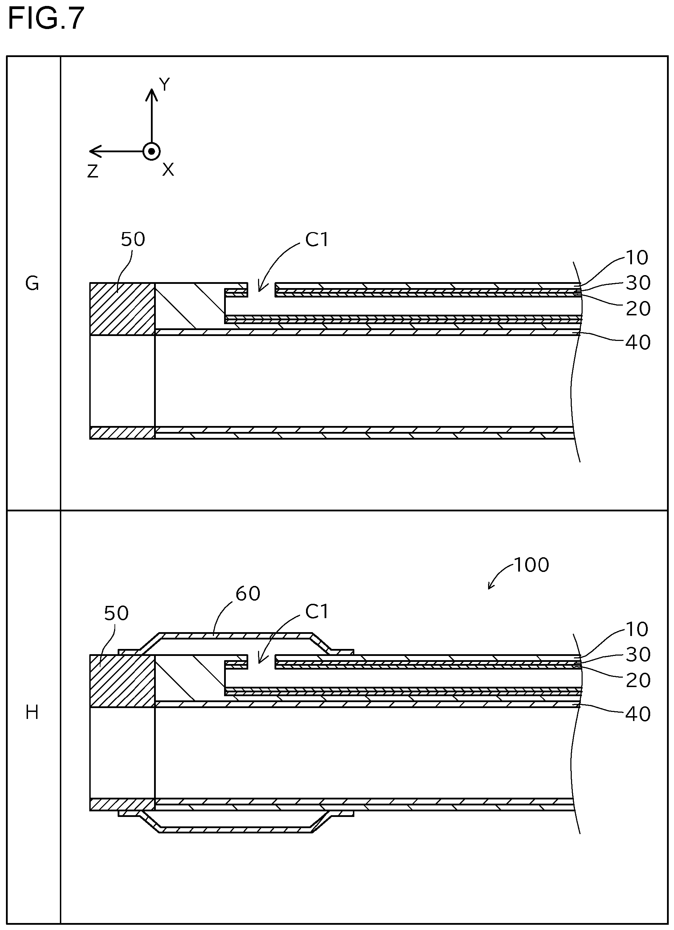

Subsequently, the distal tip 50 is joined to the distal end of the outer layer 10 e.g. by fusion bonding as illustrated in column G of FIG. 7, and the balloon 60 is joined to the outer layer 10 and the distal tip 50 e.g. by fusion bonding as illustrated in column H of FIG. 7. The Y connector 80 is attached to the proximal end portion of the outer layer 10. For example, the manufacture method as described above makes it possible to manufacture the balloon catheter 100 configured as described above.

### A-4. Effect of the First Embodiment:

As explained above, the balloon catheter 100 according to the first embodiment includes the first inner layer tube 20, the outer layer 10, the balloon 60, and the adhesive layer 30. The outer layer 10 covers the outer peripheral surface of the first inner layer tube 20. The balloon 60 covers the outer peripheral surface of the outer layer 10. The inner space 61 of the balloon 60 and the inner cavity 21 of the first inner layer tube 20 are in communication with each other. The adhesive layer 30 is placed between the first inner layer tube 20 and the outer layer 10 to bond them.

In this way, in the balloon catheter 100 according to the first embodiment, the inner cavity 21 of the first inner layer tube 20 is in communication with the inner space 61 of the balloon 60 to serve as the expansion medium lumen L1. The first inner layer tube 20 is bonded to the outer layer 10 via the adhesive layer 30. Thus, if a negative pressure is generated in the expansion medium lumen L1 due to discharge of the expansion medium through the expansion medium lumen L1, the first inner layer tube 20 can be prevented from being detached from the outer layer 10 and crushed. Consequently, the balloon catheter 100 according to the first embodiment makes it possible to suppress decrease in a cross-sectional area of the expansion medium lumen L1 due to the detachment and crushing of the first inner layer tube 20 and to achieve smooth discharge of the expansion medium through the expansion medium lumen L1.

In the balloon catheter 100 according to the first embodiment, the adhesive layer 30 has a hardness lower than hardnesses of both at least a part of the outer layer 10 and at least a part of the first inner layer tube 20. Thus, the first inner layer tube 20 can be suitably bonded to the outer layer 10 via the adhesive layer 30. Consequently, the balloon catheter 100 according to the first embodiment makes it possible to effectively suppress detachment of the first inner layer tube 20 from the outer layer 10 and to achieve smoother discharge of the expansion medium through the expansion medium lumen L1.

In the balloon catheter 100 according to the first embodiment, the first inner layer tube 20 has a hardness higher than that of the outer layer 10. Thus, if a negative pressure is generated in the expansion medium lumen L1, the first inner layer tube 20 can be effectively prevented from being crushed. Consequently, the balloon catheter 100 according to the first embodiment makes it possible to effectively suppress crushing of the first inner layer tube 20 and to achieve smoother discharge of the expansion medium through the expansion medium lumen L1.

In the balloon catheter 100 according to the first embodiment, the outer layer 10 includes the first outer layer 11, and the second outer layer 12 placed on the proximal end side relative to the first outer layer 11, and the first outer layer 11 has a hardness lower than that of the second outer layer 12. In the balloon catheter 100 according to the first embodiment, the distal end portion of the balloon catheter 100 can be made relatively softer for improving its followability for a body lumen, while the proximal end portion of the balloon catheter 100 can be made relatively harder for improving its pushability.

In the balloon catheter 100 according to the first embodiment, the adhesive layer 30 has a hardness lower than hardnesses of all of the first outer layer 11, the second outer layer 12, and the first inner layer tube 20. Thus, the adhesive layer 30 can improve the bonding of the first inner layer tube 20 to both the first outer layer 11 and the second outer layer 12. Consequently, the balloon catheter 100 according to the first embodiment makes it possible to effectively suppress detachment of the inner layer tube 20 from the outer layer 10 including the first outer layer 11 and the second outer layer 12 and to achieve smoother discharge of the expansion medium through the expansion medium lumen L1.

In the balloon catheter 100 according to the first embodiment, the first inner layer tube 20 has a hardness higher than hardnesses of both the first outer layer 11 and the second outer layer 12. Thus, if a negative pressure is generated in the expansion medium lumen L1, the first inner layer tube 20 can be more effectively prevented from being crushed. Consequently, the balloon catheter 100 according to the first embodiment makes it possible to effectively suppress crushing of the first inner layer tube 20 and to achieve smoother discharge of the expansion medium through the expansion medium lumen L1.

The method of manufacturing the balloon catheter 100 according to the first embodiment includes: a step of preparing the first inner layer tube 20 and placing the material 30X for the adhesive layer 30 on the outer peripheral surface of the first inner layer tube 20; a step of fabricating the precursor 100X by covering the first inner layer tube 20 with the material 10X for the outer layer 10; and a step of forming the outer layer 10 by heating the precursor 100X to melt the material 10X for the outer layer 10 and furthermore forming the adhesive layer 30 between the first inner layer tube 20 and the outer layer 10 by melting the material 30X for the adhesive layer 30. The method of manufacturing the balloon catheter 100 according to the first embodiment can facilitate the manufacture of the balloon catheter 100 with the first inner layer tube 20 bonded to the outer layer 10 via the adhesive layer 30.

### B. Second Embodiment:

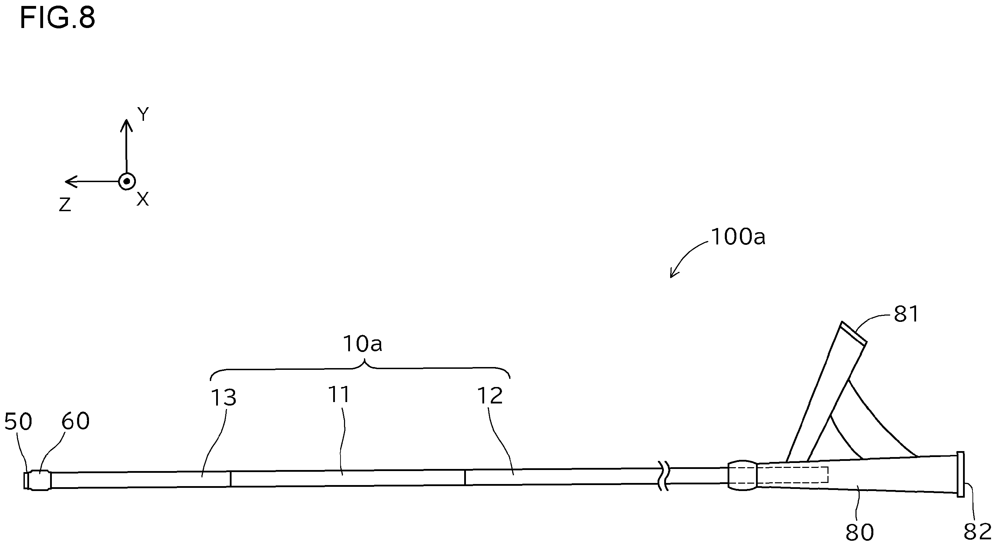

FIG. 8 is an explanatory view illustrating a side configuration of a balloon catheter 100a according to the second embodiment. Hereinafter, in the configuration of the balloon catheter 100a according to the second embodiment, the same components as in the balloon catheter 100 according to the first embodiment described above are marked with the same symbols as in the first embodiment, and their explanations are omitted as appropriate.

In the balloon catheter 100a according to the second embodiment, an outer layer 10a has, in addition to the first outer layer 11 and the second outer layer 12, a third outer layer 13 placed on the distal end side relative to the first outer layer 11. The third outer layer 13 is connected to the distal end portion of the first outer layer 11 so as to be coaxial with the first outer layer 11.

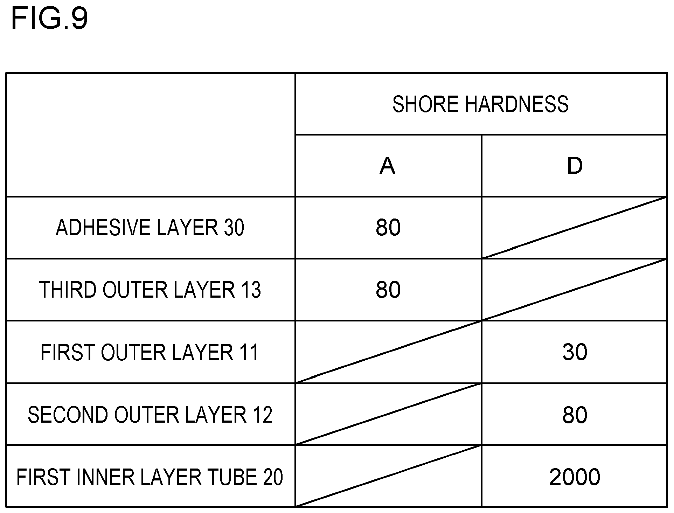

FIG. 9 is an explanatory diagram presenting a hardness of each member constituting the balloon catheter 100a according to the second embodiment. As illustrated in FIG. 9, in the balloon catheter 100a according to the second embodiment, the adhesive layer 30 has a shore hardness (A) of 80, the third outer layer 13 has a shore hardness (A) of 80, the first outer layer 11 has a shore hardness (D) of 30, the second outer layer 12 has a shore hardness (D) of 80, and the first inner layer tube 20 has a shore hardness (D) of 2000. That means, in the balloon catheter 100a according to the second embodiment, the hardnesses of the five members are set in an ascending order of the adhesive layer 30 and the third outer layer 13, the first outer layer 11, the second outer layer 12, and the first inner layer tube 20. Such a hardness relationship can be achieved e.g. by forming the adhesive layer 30 and the third outer layer 13 from a urethane resin, the first outer layer 11 from a mixed material of a urethane resin and a nylon resin, the second outer layer 12 from a nylon resin, and the first inner layer tube 20 from PEEK.

In the balloon catheter 100a according to the second embodiment, since the hardness relationship between each member is as described above, the following relationships (1) to (4) are established.
(1) The adhesive layer 30 has a hardness lower than hardnesses of both at least a part of the outer layer 10a and at least a part of the first inner layer tube 20.
(2) The first inner layer tube 20 has a hardness higher than that of the outer layer 10a.
(3) The first outer layer 11 has a hardness lower than that of the second outer layer 12.
(4) The third outer layer 13 has a hardness lower than that of the first outer layer 11.

The balloon catheter 100a according to the second embodiment includes the first inner layer tube 20, the outer layer 10a, the balloon 60, and the adhesive layer 30, similarly to the balloon catheter 100 according to the first embodiment described above. The outer layer 10a covers the outer peripheral surface of the first inner layer tube 20. The balloon 60 covers the outer peripheral surface of the outer layer 10a. The inner space 61 of the balloon 60 and the inner cavity 21 of the first inner layer tube 20 are in communication with each other. The adhesive layer 30 is placed between the first inner layer tube 20 and the outer layer 10a to bond them. Thus, similarly to the balloon catheter 100 according to the first embodiment described above, in the balloon catheter 100a according to the second embodiment, the first inner layer tube 20 can be prevented from being detached from the outer layer 10a and crushed if a negative pressure is generated in the expansion medium lumen L1 due to discharge of the expansion medium through the expansion medium lumen L1, decrease in the cross-sectional area of the expansion medium lumen L1 due to the detachment and crushing of the first inner layer tube 20 can be suppressed, and the expansion medium can be smoothly discharged through the expansion medium lumen L1.

In the balloon catheter 100a according to the second embodiment, the outer layer 10a includes the third outer layer 13, the first outer layer 11 placed on the proximal end side relative to the third outer layer 13, and the second outer layer 12 placed on the proximal end side relative to the first outer layer 11, and the third outer layer 13 has a hardness lower than that of the first outer layer 11 and the first outer layer 11 has a hardness lower than that of the second outer layer 12. Thus, in the balloon catheter 100a according to the second embodiment, the distal end portion of the balloon catheter 100a can be made relatively softer for improving its followability for a body lumen, while the proximal end portion of the balloon catheter 100a can be made relatively harder for improving its pushability, and furthermore a stiffness gap along the length direction of the balloon catheter 100a can be reduced to improve a kink resistance.

### C. Modification:

The technique disclosed in this specification is not limited to the embodiments described above but can be modified in various forms without departing from the claims, and, for example, the following modifications can be made.

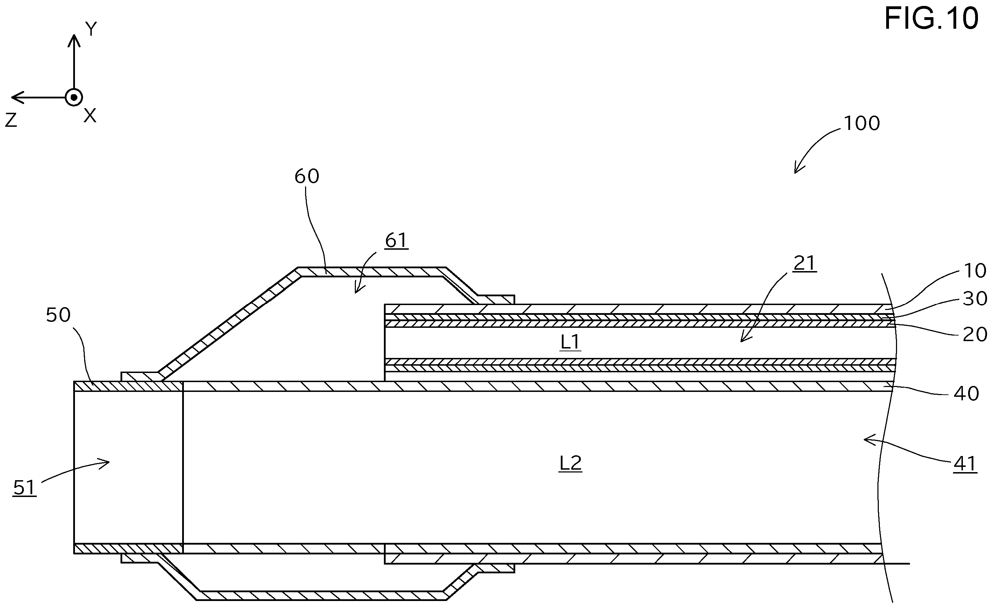

The configuration of the balloon catheter 100 according to the above embodiment is merely an example and can be variously modified. For example, in the above embodiment, although the inner space 61 of the balloon 60 and the inner cavity 21 (expansion medium lumen L1) of the first inner layer tube 20 are in communication with each other through the communication channel C1, the inner space 61 of the balloon 60 and the inner cavity 21 of the first inner layer tube 20 may be in direct contact with each other like the modification illustrated in FIG. 10. In the modification illustrated in FIG. 10, the inner cavity 21 of the first inner layer tube 20 is in communication with the inner space 61 of the balloon 60 on the distal end of the first inner layer tube 20.

In the above embodiment, although the outer layer 10 includes the first outer layer 11 and the second outer layer 12 (or the first outer layer 11, the second outer layer 12, and the third outer layer 13) aligned in an extending direction and having different hardnesses, the outer layer 10 may include four or more portions having different hardnesses, or may have a uniform hardness over the entire length. The outer and inner diameters of each portion of the outer layer 10 in the extending direction may be uniform or ununiform.

In the above embodiment, although the first inner layer tube 20 has a uniform hardness over the entire length, the first inner layer tube 20 may include a plurality of portions aligned in the extending direction and having different hardnesses. The outer and inner diameters of each portion of the first inner layer tube 20 in the extending direction may be uniform or ununiform.

The material for each member according to the above embodiment is merely an example and can be variously modified. For example, in the above embodiment, the outer layer 10, the first inner layer tube 20, the second inner layer tube 40, the distal tip 50, and other members may be made of another material (e.g. metal, carbon fiber, ceramics) instead of the resin.

The hardness values of each member and the hardness level relationship therebetween in the above embodiment are merely examples and can be variously modified. For example, in the first embodiment, although the first outer layer 11 has the hardness lower than that of the second outer layer 12, the first outer layer 11 may have a hardness higher than that of the second outer layer 12. Similarly, in the second embodiment, although the third outer layer 13 has a hardness lower than that of the first outer layer 11 and the first outer layer 11 has a hardness lower than that of the second outer layer 12, the third outer layer 13 may have a hardness higher than that of the first outer layer 11 and the first outer layer 11 may have a hardness higher than that of the second outer layer 12. Also in the first embodiment, although the adhesive layer 30 has a hardness lower than that of both the first outer layer 11 and second outer layer 12, the adhesive layer 30 may have a hardness lower than that of either one of the first outer layer 11 and second outer layer 12 and equal to or higher than that of the other one. Similarly, in the second embodiment, although the adhesive layer 30 has a hardness equal to the hardness of the third outer layer 13 and lower than that of both the first outer layer 11 and second outer layer 12, the adhesive layer 30 may have a hardness lower than that of either one of the first outer layer 11 and second outer layer 12 and equal to or higher than that of the other one and higher than that of the third outer layer 13.

The method of manufacturing the balloon catheter 100 according to the above embodiment is merely an example and can be variously modified. For example, in the above embodiment, although the joining of the distal tip 50 is performed after the removal of the distal end portion of the first inner layer tube 20 and the formation of the communication channel C1, the joining of the distal tip 50 may be performed before the removal of the distal end portion of the first inner layer tube 20 and the formation of the communication channel C1.

In the above embodiment, although the balloon catheter 100 is a so-called over-the-wire type balloon catheter, the technique disclosed in this specification is also applicable to a so-called rapid exchange (RX) type balloon catheter. In the above embodiment, although the balloon catheter 100 is a balloon guiding catheter having the device lumen L2 for guiding a device, the technique disclosed in this specification is also applicable to a balloon catheter that does not have a device lumen for guiding a device other than a guide wire but has a guide wire lumen through which a guide wire is inserted.

### DESCRIPTION OF REFERENCE NUMERALS

10: Outer layer
11: First outer layer
12: Second outer layer
13: Third outer layer
20: First inner layer tube
21: Inner cavity
30: Adhesive layer
40: Second inner layer tube
41: Inner cavity
50: Distal tip
51: Inner cavity
60: Balloon
61: Inner space
80: Y connector
81: Expansion medium port
82: Device port
100: Balloon catheter
C1: Communication channel
L1: Expansion medium lumen
L2: Device lumen

## Claims

1. A balloon catheter (100) comprising:
a first inner layer tube (20);
a second inner layer tube (40);
an outer layer (10) covering an outer peripheral surface of the first inner layer tube (20) and an outer peripheral surface of the second inner layer tube (40);
a balloon (60) covering an outer peripheral surface of the outer layer (10) and having an inner space (61) in communication with an inner cavity (21) of the first inner layer tube (20), and
an adhesive layer (30) placed between the first inner layer tube (20) and the outer layer (10), wherein
the adhesive layer (30) has a hardness lower than any of hardness of at least a part of the outer layer (10) and hardness of at least a part of the first inner layer tube (20).

2. The balloon catheter (100) according to claim 1, wherein
the first inner layer tube (20) has a hardness higher than that of the outer layer (10).

3. The balloon catheter (100) according to claim 1 or 2, wherein
the outer layer (10) includes:
a first outer layer (11); and
a second outer layer (12) placed on a proximal end side relative to the first outer layer (11), and
the first outer layer (11) has a hardness lower than that of the second outer layer (12).

4. The balloon catheter according to claim 3, wherein
the adhesive layer (30) has a hardness lower than hardnesses of all of the first outer layer (11), the second outer layer (12), and the first inner layer tube (20).

5. The balloon catheter according to claim 3 or 4, wherein
the first inner layer tube (20) has a hardness higher than any of hardness of the first outer layer (11) and hardness of the second outer layer (12).

6. The balloon catheter according to any one of claims 1 to 5, wherein
an outer diameter of the first inner layer tube (20) is smaller than an outer diameter of the second inner layer tube (40).

7. The balloon catheter according to any one of claims 1 to 6, wherein
the second inner layer tube (40) is directly bonded to the outer layer (10).

8. The balloon catheter according to any one of claims 1 to 7, wherein
a tip opening of the first inner layer tube (20) is covered by the outer layer (10), and
the inner cavity (21) of the first inner layer tube (20) communicates with the inner space (61) of the balloon (60) via a radially extending communication hole (C1).

9. A method of manufacturing a balloon catheter (100) comprising a first inner layer tube (20), a second inner layer tube (40), an outer layer (10) covering an outer peripheral surface of the first inner layer tube (20) and an outer peripheral surface of the second inner layer tube (40), and a balloon (60) covering an outer peripheral surface of the outer layer (10) and having an inner space (61) in communication with an inner cavity (21) of the first inner layer tube (20), wherein
the method comprises:
preparing the first inner layer tube (20) and disposing a material (30X) for an adhesive layer (30) on an outer peripheral surface of the first inner layer tube (20);
fabricating a precursor (100X) by covering the first inner layer tube (20) with a material (10X) for the outer layer (10); and
heating the precursor (100X) to melt the material (10X) for the outer layer (10) to form the outer layer (10) and furthermore to melt the material (30X) for the adhesive layer (30) to form the adhesive layer (30) between the first inner layer tube (20) and the outer layer (10), wherein
the adhesive layer (30) has a hardness lower than any of hardness of at least a part of the outer layer (10) and hardness of at least a part of the first inner layer tube (20).

## Patentansprüche

1. Ballonkatheter (100), mit:
einem ersten inneren Schicht-Rohr (20);
einem zweiten inneren Schicht-Rohr (40);
einer Außenschicht (10), die eine Außenumfangsfläche des ersten inneren Schicht-Rohrs (20) und eine Außenumfangsfläche des zweiten inneren Schicht-Rohrs (40) bedeckt;
einem Ballon (60), der eine Außenumfangsfläche der Außenschicht (10) bedeckt und einen Innenraum (61) aufweist, der mit einem Innenhohlraum (21) des ersten inneren Schicht-Rohrs (20) in Verbindung steht, und
einer Klebeschicht (30), die zwischen dem ersten inneren Schicht-Rohr (20) und der Außenschicht (10) angeordnet ist, wobei
die Klebeschicht (30) eine Härte aufweist, die geringer ist die Härte zumindest eines Teils der Außenschicht (10) und die Härte zumindest eines Teils des ersten inneren Schicht-Rohrs (20).

2. Ballonkatheter (100) nach Anspruch 1, wobei
das erste innere Schicht-Rohr (20) eine Härte aufweist, die höher ist als die der Außenschicht (10).

3. Ballonkatheter (100) nach Anspruch 1 oder 2, wobei
die Außenschicht (10) enthält:
eine erste Außenschicht (11); und
eine zweite Außenschicht (12), die relativ zur ersten Außenschicht (11) auf der Seite des proximalen Endes angeordnet ist, und
die erste Außenschicht (11) eine geringere Härte aufweist als die zweite Außenschicht (12).

4. Ballonkatheter nach Anspruch 3, wobei
die Klebeschicht (30) eine Härte aufweist, die geringer ist als die Härte der ersten Außenschicht (11), der zweiten Außenschicht (12) und des ersten inneren Schicht-Rohrs (20).

5. Ballonkatheter nach Anspruch 3 oder 4, wobei
das erste innere Schicht-Rohr (20) eine Härte aufweist, die höher ist als die Härte der ersten Außenschicht (11) und die Härte der zweiten Außenschicht (12).

6. Ballonkatheter nach einem der Ansprüche 1 bis 5, wobei
der Außendurchmesser des ersten inneren Schicht-Rohrs (20) kleiner ist als der Außendurchmesser des zweiten inneren Schicht-Rohrs (40).

7. Ballonkatheter nach einem der Ansprüche 1 bis 6, wobei
das zweite innere Schicht-Rohr (40) direkt mit der Außenschicht (10) verbunden ist.

8. Ballonkatheter nach einem der Ansprüche 1 bis 7, wobei
eine Spitzenöffnung des ersten inneren Schicht-Rohrs (20) von der Außenschicht (10) bedeckt ist und
der Innenhohlraum (21) des ersten inneren Schicht-Rohrs (20) über ein sich radial erstreckendes Verbindungsloch (C1) mit dem Innenraum (61) des Ballons (60) in Verbindung steht.

9. Verfahren zur Herstellung eines Ballonkatheters (100) mit einem ersten inneren Schicht-Rohr (20), einem zweiten inneren Schicht-Rohr (40), einer Außenschicht (10), die eine Außenumfangsfläche des ersten inneren Schicht-Rohrs (20) und eine Außenumfangsfläche des zweiten inneren Schicht-Rohrs (40) bedeckt, und einem Ballon (60), der eine Außenumfangsfläche der Außenschicht (10) bedeckt und einen Innenraum (61) aufweist, der mit einem Innenhohlraum (21) des ersten inneren Schicht-Rohrs (20) in Verbindung steht, wobei
das Verfahren umfasst:
Bereitstellen des ersten inneren Schicht-Rohrs (20) und das Aufbringen eines Materials (30X) für eine Klebeschicht (30) auf eine Außenumfangsfläche des ersten inneren Schicht-Rohrs (20);
Herstellen eines Vorläufers (100X) durch Bedecken des ersten inneren Schicht-Rohrs (20) mit einem Material (10X) für die Außenschicht (10); und
Erhitzen des Vorläufers (100X), um das Material (10X) für die Außenschicht (10) zu schmelzen und die Außenschicht (10) zu bilden, und ferner um das Material (30X) für die Klebeschicht (30) zu schmelzen und die Klebeschicht (30) zwischen dem ersten inneren Schicht-Rohr (20) und der Außenschicht (10) zu bilden, wobei
die Klebeschicht (30) eine Härte aufweist, die geringer ist als die Härte zumindest eines Teils der Außenschicht (10) und die Härte zumindest eines Teils des ersten inneren Schicht-Rohrs (20).

## Revendications

1. Cathéter à ballonnet (100) comprenant :
un premier tube de couche intérieure (20) ;
un deuxième tube de couche intérieure (40) ;
une couche extérieure (10) recouvrant une surface périphérique extérieure du premier tube de couche intérieure (20) et une surface périphérique extérieure du deuxième tube de couche intérieure (40) ;
un ballonnet (60) recouvrant une surface périphérique extérieure de la couche extérieure (10) et présentant un espace intérieur (61) en communication avec une cavité intérieure (21) du premier tube de couche intérieure (20), et
une couche adhésive (30) placée entre le premier tube de couche intérieure (20) et la couche extérieure (10), dans lequel
la couche adhésive (30) présente une dureté inférieure à la dureté d'au moins une partie de la couche extérieure (10) et à la dureté d'au moins une partie du premier tube de couche intérieure (20).

2. Cathéter à ballonnet (100) selon la revendication 1, dans lequel
le premier tube de couche intérieure (20) présente une dureté supérieure à celle de la couche extérieure (10).

3. Cathéter à ballonnet (100) selon la revendication 1 ou 2, dans lequel
la couche extérieure (10) inclut :
une première couche extérieure (11) ; et
une deuxième couche extérieure (12) placée sur un côté d'extrémité proximale par rapport à la première couche extérieure (11), et
la première couche extérieure (11) présente une dureté inférieure à celle de la deuxième couche extérieure (12).

4. Cathéter à ballonnet selon la revendication 3, dans lequel
la couche adhésive (30) présente une dureté inférieure à celle de tous parmi la première couche extérieure (11), la deuxième couche extérieure (12) et le premier tube de couche intérieure (20).

5. Cathéter à ballonnet selon la revendication 3 ou 4, dans lequel
le premier tube de couche intérieure (20) présente une dureté supérieure à celle de la première couche extérieure (11) et à celle de la deuxième couche extérieure (12).

6. Cathéter à ballonnet selon l'une quelconque des revendications 1 à 5, dans lequel
le diamètre extérieur du premier tube de couche intérieure (20) est plus petit que le diamètre extérieur du deuxième tube de couche intérieure (40).

7. Cathéter à ballonnet selon l'une quelconque des revendications 1 à 6, dans lequel
le deuxième tube de couche intérieure (40) est directement lié à la couche extérieure (10).

8. Cathéter à ballonnet selon l'une quelconque des revendications 1 à 7, dans lequel
une ouverture terminale du premier tube de couche intérieure (20) est recouverte par la couche extérieure (10), et
la cavité intérieure (21) du premier tube de couche intérieure (20) communique avec l'espace intérieur (61) du ballonnet (60) via un trou de communication s'étendant radialement (C1).

9. Procédé de fabrication d'un cathéter à ballonnet (100) comprenant un premier tube de couche intérieure (20), un deuxième tube de couche intérieure (40), une couche extérieure (10) recouvrant la surface périphérique extérieure du premier tube de couche intérieure (20) et la surface périphérique extérieure du deuxième tube de couche intérieure (40), et un ballonnet (60) recouvrant une surface périphérique extérieure de la couche extérieure (10) et présentant un espace intérieur (61) en communication avec une cavité intérieure (21) du premier tube de couche intérieure (20), dans lequel
le procédé comprend :
la préparation du premier tube de couche intérieure (20) et la disposition d'un matériau (30X) pour une couche adhésive (30) sur une surface périphérique extérieure du premier tube de couche intérieure (20) ;
la fabrication d'un précurseur (100X) en recouvrant le premier tube de couche intérieure (20) d'un matériau (10X) pour la couche extérieure (10) ; et
le chauffage du précurseur (100X) pour faire fondre le matériau (10X) destiné à la couche extérieure (10) afin de former ladite couche extérieure (10) et, en outre, faire fondre le matériau (30X) destiné à la couche adhésive (30) afin de former ladite couche adhésive (30) entre le premier tube de couche intérieure (20) et la couche extérieure (10), dans lequel
la couche adhésive (30) présente une dureté inférieure à la dureté d'au moins une partie de la couche extérieure (10) et à la dureté d'au moins une partie du premier tube de couche intérieure (20).
